(19) Europäisches Patentamt
European Patent Office
Office européen des brevets

(11) Publication number: **0 070 075 B2**

# (12) NEW EUROPEAN PATENT SPECIFICATION

(45) Date of publication of the new patent specification: **04.11.92 Bulletin 92/45**

(51) Int. Cl.$^5$: **C11D 1/83,** C11D 1/66, C11D 1/72

(21) Application number: **82200869.4**

(22) Date of filing: **12.07.82**

## (54) Foaming dishwashing liquid compositions.

The file contains technical information submitted after the application was filed and not included in this specification

(30) Priority: **26.04.82 US 371694**
**13.07.81 US 282976**

(43) Date of publication of application: **19.01.83 Bulletin 83/03**

(45) Publication of the grant of the patent: **21.01.87 Bulletin 87/04**

(45) Mention of the opposition decision: **04.11.92 Bulletin 92/45**

(84) Designated Contracting States: **AT BE CH DE FR GB IT LI NL**

(56) References cited:
**FR-A- 2 093 790**

(56) References cited:
**US-A- 3 721 633**
**US-A- 3 839 318**
**US-A- 4 154 706**
**US-A- 4 240 921**
**Rohm & Haas, Technical Bulletin, Triton CG-110**

(73) Proprietor: **THE PROCTER & GAMBLE COMPANY**
**301 East Sixth Street**
**Cincinnati Ohio 45202 (US)**

(72) Inventor: **Cook, Thomas Edward**
**2852 Westonridge Dr.**
**Cincinnati, OH 45239 (US)**
Inventor: **Llenado, Ramon Aquillon**
**8090 Pepper Pike**
**West Chester, OH 45069 (US)**

(74) Representative: **Canonici, Jean-Jacques et al**
**Procter & Gamble European Technical Center N.V. Temselaan 100**
**B-1853 Strombeek-Bever (BE)**

EP 0 070 075 B2

## Description

This invention relates to dishwashing liquid detergent compositions which provide controllable aqueous foams.

Alkylpolyglycosides which are surfactants have been disclosed in U.S.Patents 3,598,865; 3,721,633 and 3,772,269. These patents also disclose processes for making alkylpolyglycoside surfactants and built liquid detergent compositions containing these surfactants. U.S. Patent 3,219,656 discloses alkylmonoglucosides and suggests their utility as foam stabilisers for other surfactants. Various polyglycoside surfactant structures and processes for making them are disclosed in U.S. Patents 2,974,134, 3,640,998, 3,839,318, 3,314,936, 3,346,558, 4,011,389, 4,223,129.

Foaming compositions containing an alkylpolysaccharide surfactant, an anionic cosurfactant and possibly an auxiliary foam booster, are described in copending published EP-A-0070076 and EP-A-0070074.

Light duty detergent compositions containing an alkylpolysaccharide, alkylbenzene sulfonate and alkylpolyethoxylate sulfate cosurfactants are described in published EP-A-0070077.

All percentages, parts and ratios used herein are by weight unless otherwise specified.

This invention relates to foaming dishwashing liquid detergents comprising 10-50% by weight of the composition of

(1) an alkyl polyglucoside of the formula $R^2O(C_nH_{2n}O)_t(Z)_x$ wherein Z is derived from glucose, $R^2$ is a hydrophobic saturated alkyl group containing from 12 to 18, preferably from 12 to 14 carbon atoms; n is 2 or 3, preferably 2, t is from 0 to 10, preferably 0; and x is from 1.5 to 4, preferably from 1.6 to 2.7, and including less than 10% unreacted fatty alcohol and less than 50% short chain alkyl polyglucoside and

(2) a mixture of aniomic cosurfactants neutralised with one or more cationic moieties consisting essentially of:

(a) from 1 to 95%, preferably from 10 to 50%, of a water-soluble alkylbenzene sulfonate in which the alkyl group contains from 10 to 13 carbon atoms, and

(b) from 5 to 99%, preferably from 50-90%, of a cosurfactant selected from the group consisting of an alkyl glyceryl ether sulfonate in which the alkyl group contains from 8 to 18 carbon atoms, an alphaolefin sulfonate in which the olefin group contains atom 10 to 18 carbon atoms, an alkyl polyethoxylate carboxylate in which the alkyl group contains from 10 to 18 carbon atoms, and the polyethoxylate chain contains from 2 to 6 ethoxylate groups, and mixtures thereof, the weight ratio (2) : (1) being from 1 : 10 to 10 : 1.

It has surprisingly been found that the cosurfactants interact with the alkylpolyglucoside surfactant of this invention to provide a relatively stable foam which is readily rinsed and in particular that gives good suds mileage and "Suds During Washing" values.

The alkylpolyglucosides are those having an alkyl hydrophobic group that is saturated and that contains from 12 to 18 carbon atoms, preferably from 12 to 16 carbon atoms, most preferably from 12 to 14 carbon atoms. The number x indicates the number of glucoside units in a particular alkylpolyglucoside surfactant. For a particular alkylpolyglucoside molecule x can only assume integral values. In any physical sample of alkypolyglucoside surfactants there will in general be molecules having different x values. The physical sample can be characterised by the average value of x and this average value can assume non-integral values. In this specification the values of x are to be understood to be average values. The hydrophobic group ($R^2$) can be attached at the 2-, 3-, or 4-positions rather than at the 1-position, (thus giving e.g. a glucosyl as opposed to a glucoside). However, attachment through the 1-position, i.e., glucosides, is preferred. In the preferred product the additional glucoside unit's are predominately attached to the previous glucoside units 2-position. Attachment through the 3-,4-, and 6-positions can also occur.

Optionnaly, and less desirably there can be a polyalkoxide chain joining the hydrophobic moiety $R^2$ and the polyglucoside chain. The preferred alkoxide moiety is ethoxide.

Suitable alkyl polyglucosides are dodecyl, tetradecyl, hexadecyl, and octadecyl, di-, tri- and tetraglucosides.

The alkylmonoglucosides are relatively less soluble in water than the higher alkylpolyglucosides. When used in admixture with alkylpolyglucosides the alkylmonoglucosides are solubilised to some extent. The use of alkylmonoglucosides in admixture with alkylpolyglucosides is a preferred mode of carrying out the invention. Suitable mixtures include coconut alkyl, di-, tri- and tetraglucosides and tallow alkyl tetraglucosides.

To prepare these compounds a long chain alcohol ($R^2OH$) can be reacted with glucose, in the presence of an acid catalyst to form the desired glucosides. Alternatively the alkylpolyglucosides can be prepared by a two step procedure in which a short chain alcohol ($C_{1-6}$) is reacted with glucose or a polyglucoside (x=2 to 4) to yield a short chain alkyl glucoside (x=1 to 4) which can in turn be reacted with a longer chain alcohol ($R^2OH$) to displace the short chain alcohol and obtain the desired alkylpolyglucoside. If this two step procedure is used, the short chain alkylglucoside content of the final alkylpolyglucoside material should be less than 50%, preferably less than 10%, more preferably less than 5%, most preferably 0% of the alkylpolyglucoside. The amount of unreacted alcohol (the free fatty alcohol content) in the desired alkylpolyglucoside surfactant is preferably less than 2% more preferably less than 0.5% by weight of the total of the alkyl polyglucoside plus unreacted alcohol. The amount of alkylmonoglucoside is 20% to 70%, preferably 30% to 60%, most preferably 30% to 50% by weight of the total of the alkylpolyglucoside. For some uses it is desirable to have the alkylmonoglucoside content less than 10%. Throughout this specification, "alkylglucoside" is used to include alkylglycosides because the stereochemistry of the saccharides moiety is changed during the preparation reaction.

Anionic cosurfactants can be selected from the group consisting of sulfonates, carboxylates and mixtures thereof. The cosurfactants are neutralised with a cationic moiety or moieties selected from the group consisting of alkali metal, e.g. sodium or potassium, alkaline earth metal, e.g. calcium or magnesium, ammonium, substituted ammonium, including mono-, di-, or tri-, ethanolammonium cations. Mixtures of cations can be desirable. The anionic cosurfactants useful in the present invention all have detergent properties and are all water soluble or dispersible in water.

One of the cosurfactants for use in this invention. is an alkylbenzene sulfonate. The alkyl group can be either saturated or unsaturated, branched or straight chain and is optionally substituted with a hydroxy group. Middle phenyl positions are generally preferred for volume of foaming in light soil conditions. However, in heavier soil conditions, phenyl attachment at the 1-or 2-position is preferred.

The preferred alkylbenzene sulfonates contain a straight alkyl chain containing from 10 to 13 carbon atoms, and the cation is sodium, potassium, ammonium, mono-, di-, or triethanolammonium, calcium or magnesium and mixtures thereof. Magnesium is the preferred cationic moiety. These same cations are preferred for other anionic surfactants and ingredients. The magnesium alkylbenzene sulfonates where the phenyl group is attached near the middle of the alkyl chain are surprisingly better than the ones with the phenyl near the end of the chain when the polysaccharide chain averages greater than 3 saccharide units. Suitable alkylbenzene sulfonates include $C_{11}$ alkylbenzene sulfonates with low 2-phenyl content.

Many other surfactants which contain sulfonate or carboxylate groups can be used in the foaming compositions of the invention. Generally the use of these latter cosurfactants produces less foam volume than does the use of the preferred cosurfactants. However, the alkylpolyglucoside surfactant stabilises the foams which are produced and allows the foams to be rinsed more quickly.

One group of cosurfactants that are of interest because of their superior detergency are the zwitterionic detergents which contain both a cationic group, either ammonium, phosphonium, sulfonium or mixtures thereof and a sulfonate or carboxylate group. Preferably there are at least four atoms separating the cationic and anionic groups. Suitable zwitterionic surfactant are disclosed in U.S.Patents 4,159,277; 3,928,251; 3,925,262, 3,929,678; 3,227,749; 3,539,521; 3,383,321, 3,390,094, and 3,239,560.

Another group of cosurfactants are the amphoteric detergents which have the same general structure as the zwitterionic surfactants but with an amine group instead of the quaternary ammonium group.

Yet other cosurfactants are the alkyl (paraffin or olefin) sulfonates, preferably with a more central hydrophilic group, containing from 6 to 30 carbon atoms. Compositions containing these cosurfactants produce the least volume of foam, if that is desired. The hydrophobic group can contain up to 10 hydroxy groups and/or ether linkages. Examples include $C_{14-15}$ paraffin sulfonates and $C_{14-16}$ olefin sulfonates.

Still another cosurfactant is a soap structure containing up to 10 ether linkages in the chain and from 1 to 4 carbon atoms between ether linkages with from 6 to 30 carbon atoms in a terminal portion containing no ether linkages.

The alkylpolyglucosides can be contaminated with less than 50% short chain alkylpolyglucosides and with less than 10%, preferably less than 2%, most preferably less than 0.5% unreacted fatty alcohol and increase the sudsing abilily of conventional sulfate detergent cosurfactants, especially alkyl sulfate and alkyl polyether sulphate cosurfactants having he formula:

$$R^3O(C_nH_{2n}O)_tSO_3M$$

wherein $R^3$ is an alkyl or hydroxyalkyl group containing from 8 to 18 carbon atoms, n is 2 or 3, t can vary from 0 to 30, and M is a cationic moiety as defined above, the cosurfactant being water soluble or dispersible.

Such compostions have improved suds mileage as compared to compositions containing only the alkyl benzene sulfonate cosurfactant and the alkylpolyglucoside surfactant.

Typical compositions for use as light duty liquid detergent compositions in washing dishes comprise from 10% to 50%, preferably from 10% to 40% of the mixture of surfactants disclosed hereinbefore and from 1% to 50% of a solvent selected from the group consisting of $C_{1-3}$ alkanols, $C_{1-3}$ alkanolamines, $C_{2-4}$ polyols, mixtures thereof, and the balance water. It is a special advantage of the compositions of this invention that they can be made in concentrated form (up to 50% by wt, of the mixture of surfactants) with only very low levels of organic solvents and without the addition of expensive hydroptropic materials. Fatty alcohols should not be used.

The compositions of this invention can utilise

other compatible ingredients, including other surfactants, in addition to the mixture of surfactants herein disclosed.

The following nonlimiting examples illustrate the foaming compositions of the present invention.

The relative volume of suds in ml is determined by the following test procedure:

100ml of the test solution at 44.5°C is placed in a 500ml graduated cylinder: the solution is agitated by repeated inversion of the graduated cylinder until the amount of suds in the cylinder does not increase with further agitation. Suds height is measured directly on the cylinder scale making allowance for the height of liquid remaining in the cylinder. The test solution is made by adding the test product to water having a hardness of 0.12 gram per liter (Ca/Mg=3/1).

Example 1

Ammonium $C_{11.2}$ linear alkyl benzene sulfonate was admixed with $C_{12}$ alkylpolyglucoside $G_{3.5}$ in a ratio of 2:1. The mixture was used at a level of 400ppm in city water. The initial suds volume was more than 300ml, but after the addition of about 1.25 grams of a standard grease soil per 200 ml of wash solution, the suds had disappeared. Substitution of a sodium $C_{12-16}$ alkyl glyceryl ether sulfonate for 25% and 40% of the mixture extended the point at which there was no suds to 1.5 and 1.75 grams of soil per 200ml of wash solution respectively.

Similar results are obtained when a sodium, potassium, ammonium, or monoethanol-ammonium $C_{12-16}$ alkylpolyethoxy$_3$ acetate, or $C_{14-16}$ olefin sulfonate or mixtures thereof is substituted for at least part of the alkyl glyceryl ether sulfonate.

Example 2

A 2:1 mixture of an ammonium $C_{11.2}$ alkyl benzene sulfonate and the $C_{12-13}$ alkylpolyglucoside (2-4) ( >2% free fatty alcohol) are tested for suds volume as described hereinbefore. The initial suds volume is good, but the SDW (Suds During Washing) grade is not a good as some premium commercial products. Substitution of between 25% and 50% of the mixture with a sodium $C_{12-16}$ alkyl glyceryl ether sulfonate, or sodium $C_{14-16}$ olefin sulfonate, or sodium $C_{12-13}$ alkyl polyethoxylate (3) acetate increases the SDW grade without lowering the initial sudsing excessively.

Known analytical techniques can be used to determine the structures of the alkylpolyglucoside surfactants herein; for example, to determine the glycosidic chain length, the amount of butyl glucoside, the free fatty alcohol content, and the level of unreacted polyglucoside. More specifically, gas or liquid chromatography can be used to determine the unreacted alcohol content and the unreacted polyglucoside content respectively. Proton nmr can be used to determine the average glucosidic chain length. The point of attachment of the hydrophilic portion of the molecule to the hydrophobic portion of the molecule can be determined by $^{13}C$ nmr.

The alkypolyglucoside surfactants are complex mixtures. Their components vary depending upon the nature of the starting materials and the reaction by which they are prepared. Analytical standards which are useful in calibrating instruments for analysing the components of a particular alkylpolyglucoside surfactant can be obtained from Calbiochem Behring Co. LaJolla, California. These standards include those for octylglucoside (Calbiochem #494559), decylglucoside (Calbiochem #252715), dodecylmaltoside (Calbiochem #3243555).

**Claims**

1. A foaming dishwashing liquid detergent composition comprising 10 to 50% (by weight of the composition) of

   (1) a mixture of anionic cosurfactants neutralised with one or more cationic moieties consisting essentially of:

      (a) from 1 to 95% by weight, of a water soluble alkylbenzene sulfonate in which the alkyl group contains from 10 to 13 carbon atoms and

      (b) from 5 to 99% by weight, of a cosurfactant selected from the group consisting of an alkyl glyceryl ether sulfonate in which the alkyl group contains from 8 to 18 carbon atoms, an alpha olefin sulfonate in which the olefin group contains from 10 to 18 carbons atoms an alkyl polyethoxylate carboxylate in which the alkyl group contains from 10 to 18 carbon atoms, and the polyethoxylate chain contains from 2 to 6 ethoxylate groups and mixtures thereof and

   (2) an alkylpolyglucoside surfactant having the formula $R^2O\ (C_nH_{2n}O)_tZ_x$ wherein Z is a moiety derived from glucose; $R^2$ is an alkyl group that contains from 12 to 18 carbon atoms; t is from 0 to 10 and x is from 1.5 to 4; n is 2 or 3 and including less than 10% fatty alcohol and less than 50% short chain alkyl polyglucosides, the weight ratio of (1):(2) being from 1:10 to 10:1

2. A composition according to claim 1 in which x is 1.6 to 2.7.

3. A composition according to any preceding claim wherein the cosurfactant (1) (b) is an alkyl glyceryl ether sulfonate and the cationic moiety is selected from the group consisting of sodium, potassium, ammonium, monoethanolammonium, diethanolammonium, triethanolammonium, calcium, magnesium and mixtures thereof.

**4.** The composition of claim 1, wherein the cosurfactant (1) (b) is an alkyl polyethoxylate carboxylate.

**5.** The composition of claim 1 wherein the cosurfactant (1) (b) is an alpha olefin sulfonate.

**6.** A dishwashing liquid detergent composition comprising from 5% to 50% by weight of the surfactant mixture of claim 1 and from 1% to 50% by weight of a solvent selected from the group consisting of $C_{1-3}$ alcohols, $C_{1-3}$ hydroxyalkylamines, $C_{2-4}$ polyols, and mixtures thereof, and the balance water.

## Patentansprüche

**1.** Shäumende, flüssige Geschirrspüldetergenszusammensetzung, enthaltend 10 % bis 50 % (bezogen auf das Gewicht der Zusammensetzung) von

(1) einem Gemisch von mit einem oder mehreren kationischen Resten neutralisierten anionischen co-grenzflächenaktiven Mitteln, das im wesentlichen aus:

(a) 1 Gew.-% bis 95 Gew.-% eines wasserlöslichen Alkylbenzolsulfonats, in welchem die Alkygruppe 10 bis 13 Kohlenstoffatome enthält, und

(b) 5 Gew.-% bis 99 Gew.-% eines co-grenzflächenaktiven Mittels, ausgewählt aus der Gruppe, bestehend aus einem Alkylglycerylethersulfonat, in welchem die Alkylgruppe 8 bis 18 Kohlenstoffatome enthält, einem α-Olefinsulfonat, in welchem die Olefingruppe 10 bis 18 Kohlenstoffatome enthält, einem Alkylpolyethoxylatcarboxylat, in welchem die Aklygruppe 10 bis 18 Kohlenstoffatome enthält, und die Polyethoxylatkette 2 bis 6 Ethoxylatgruppen enthält, und Mischungen davon, besteht, und

(2) einem Alkylpolyglucosid-grenzflächenaktiven Mittel der Formel $R^2O(C_nH_{2n}O)_tZ_x$, worin Z ein sich aus Glucose herleitender Rest ist; $R^2$ eine Alkylgruppe mit 12 bis 18 Kohlenstoffatomen darstellt; t von 0 bis 10 beträgt und x von 1,5 bis 4 ist; n 2 oder 3 ist, und welches weniger als 10 % Fettalkohol und weniger als 50 % an kurzkettigem Alkylpolyglucosid enthält, wobei das Gewichtsverhältnis von (1):(2) von 1:10 bis 10:1 beträgt.

**2.** Eine Zusammensetzung nach Anspruch 1, wobei x von 1,6 bis 2,7 ist.

**3.** Eine Zusammensetzung nach einem der vorstehenden Ansprüche, wobei das co-grenzflächenaktive Mittel (1) (b) ein Alkylglycerylethersulfonat ist, und der kationische Rest aus der Gruppe ausgewählt ist, die aus Natrium, Kalium, Ammonium, Monoethanolammonium, Diethanolammonium, Triethanolammoniumn, Calcium, Magnesium, und Mischungen davon, besteht.

**4.** Die Zusammensetzung des Anspruchs 1, wobei das co-grenzflächenaktive Mittel (1) (b) ein Alkylpolyethoxylatcarboxylat ist.

**5.** Die Zusammensetzung des Anspruchs 1, wobei das co-grenzflächenaktive Mittel (1) (b) ein α-Olefinsulfonat ist.

**6.** Eine flüssige Geschirrspüldetergenszusammensetzung, enthaltend 5 Gew.-% bis 50 Gew.-% des Gemisches grenzflächenaktiver Mittel nach Anspruch 1 und 1 Gew.-% bis 50 Gew.-% eines Lösungsmittels, das aus der aus $C_{1-3}$-Alkoholen, $C_{1-3}$-Hydroxyalkylaminen, $C_{2-4}$-Polyolen, und Mischungen davon, bestehenden Gruppe ausgewählt ist, und als Rest Wasser.

## Revendications

**1.** Composition détergente liquide moussante pour lavage de la vaisselle comprenant 10 à 50 % (en poids de la composition) de

(1) un mélange de co-agents tensio-actifs anioniques neutralisés par un ou plusieurs groupes cationiques constitués essentiellement de :

(a) 1 à 95 % en poids d'un alkyl benzène sulfonate soluble dans l'eau dans lequel le groupe alkyle contient 10 à 13 atomes de carbone et

(b) 5 à 99 % en poids d'un co-agent tensio-actif choisi parmi le groupe consitué d'un alkyl glycéryl éther sulfonate dans lequel le groupe alkyle contient 8 à 18 atomes de carbone, d'un alpha oléfine sulfonate dans lequel le groupe oléfine contient 10 à 18 atomes de carbone, d'un alkyl polyéthoxylate carboxylate dans lequel le groupe alkyle contient 10 à 18 atomes de carbone et la chaîne de polyéthoxylate contient 2 à 6 groupes éthoxylate et de leurs mélanges et

(2) un agent tensio-actif à base d'alkyl polyglucoside ayant pour formule

$R^2O(C_nH_{2n}O)_tZ_x$

dans laquelle Z est un radical dérivé du glucose; $R^2$ est un groupe alkyl qui contient 12 à 18 atomes de carbone; t est égal à 0 à 10 et x est égal à 1,5 à 4; n est égal à 2 ou 3, et comprenant moins de 10 % d'alcool gras et moins de 50 % d'alkyl polyglucoside à chaîne courte, le rapport pondéral de (1) : (2) étant de 1 : 10 à 10 : 1.

2. Composition selon la revendication 1, dans laquelle x est égal à 1,6 à 2,7.

3. Composition selon l'une quelconque des revendications précédentes, dans laquelle le co-agent tensio-actif (1) (b) est un alkyl glycéryl éther sulfonate et le groupe cationique est choisi parmi le groupe constitué du sodium, du potassium, de l'ammonium, du monoéthanolammonium, du diéthanolammonium, du triéthanolammonium, du calcium, du magnésium et de leurs mélanges.

4. Composition selon la revendication 1, dans laquelle le co-agent tensio-actif (1) (b) est un alkyl polyéthoxylate carboxylate.

5. Composition selon la revendication 1, dans laquelle le co-agent tensio-actif (1) (b) est un alpha oléfine sulfonate.

6. Composition détergente liquide pour le lavage de la vaisselle comprenant 5 à 50 % en poids du mélange d'agents tensio-actifs de la revendication 1 et 1 à 50 % en poids d'un solvant choisi dans le groupe constitué des alcools en $C_{1-3}$, des hydroxyalkylamines en $C_{1-3}$, des polyols en $C_{2-4}$ et de leurs mélanges, le restant étant constitué d'eau.